**Europäisches Patentamt**

**European Patent Office**

**Office européen des brevets**

(19)

(11) Veröffentlichungsnummer : **0 359 700 B1**

# EUROPÄISCHE PATENTSCHRIFT

(12)

(45) Veröffentlichungstag der Patentschrift :
**19.11.92 Patentblatt 92/47**

(21) Anmeldenummer : **89810531.7**

(22) Anmeldetag : **13.07.89**

(51) Int. Cl.⁵ : **A61F 2/34**

(54) **Zweiteilige Hüftgelenkspfanne.**

(30) Priorität : **24.08.88 CH 3145/88**

(43) Veröffentlichungstag der Anmeldung :
**21.03.90 Patentblatt 90/12**

(45) Bekanntmachung des Hinweises auf die
Patenterteilung :
**19.11.92 Patentblatt 92/47**

(84) Benannte Vertragsstaaten :
**AT DE FR GB IT**

(56) Entgegenhaltungen :
**EP-A- 0 038 903**
**EP-A- 0 190 981**
**EP-A- 0 242 633**
**EP-A- 0 242 719**

(56) Entgegenhaltungen :
**EP-A- 0 262 379**
**WO-A-85/00284**
**CH-A- 652 913**
**FR-A- 2 592 787**
**GB-A- 2 069 338**
**US-A- 4 662 891**

(73) Patentinhaber : **GEBRÜDER SULZER**
**AKTIENGESELLSCHAFT**
**Zürcherstrasse 9**
**CH-8401 Winterthur (CH)**

(72) Erfinder : **Frey, Otto, Dr.**
**Wallrütistrasse 56**
**CH-8400 Winterthur (CH)**
Erfinder : **Koch, Rudolf**
**Oberdorfstrasse 229**
**CH-8267 Berlingen (CH)**

EP 0 359 700 B1

## Beschreibung

Die Erfindung betrifft eine zweiteilige Hüftgelenkspfanne zur zementfreien Verankerung im Becken, bestehend aus einer einseitig geschlitzten, kegelstumpfförmigen Hülse und einem die eigentliche Pfannenschale enthaltenden, im Passitz in den Innenhohlraum der Hülse einpressbaren Einsatz, wobei die aus Metall gefertigte Hülse in Umfangsrichtung Bereiche grösserer Wandstärke alternierend mit solchen geringerer Wandstärke aufweist.

Eine Hüftgelenkspfanne der genannten Art ist beispielsweise aus der CH-A-652 913 bekannt. Die zementfreie Fixierung dieser bekannten Pfanne, bei der Hülse und Einsatz vorzugsweise aus Kunststoff bestehen, erfolgt durch Aufweiten der Hülse beim Einpressen des Einsatzes. Dieses Aufweiten geschieht dabei vorzugsweise in dem dem Schlitz diametral gegenüberliegenden Bereich des Umfangs der Hülse. Soll die Hülse, wie in letzter Zeit üblich, aus Metall, beispielsweise aus Reintitan oder einer Titanlegierung, gefertigt werden, so ergeben sich mit der Aufweitung, die - beispielsweise zur Vereinfachung von Reoperationen - elastisch bleiben soll, Schwierigkeiten, da das Metall im Aufweitungsbereich überelastisch gedehnt und bleibend verformt wird.

Die Bereiche geringerer Wandstärke bestehen bei der vorstehend beschriebenen, bekannten Konstruktion lediglich aus entlang einer Mantellinie verlaufenden, schmalen Rillen, die als Verdrehsicherungen dienen; sie beeinflussen die "Aufweit"-Elastizität der Hülse praktisch nicht.

Aus der EP-A-0 038 903 ist eine einteilige, ungeschlitzte Hüftgelenkspfanne bekannt, die auf dem Umfang ihres Aussenmantels alternierend Bereiche geringerer Wandstärke und solche grösserer Wandstärke aufweist; aufgrund dieser Ausbildung des Aussenmantels soll diese Pfanne des elastischen Verformungen des Beckens in gewissem Umfang nachgeben. Da jedoch ihr Aussenmantel nicht geschlitzt ist, existiert hier auch kein, auf einen engen Umfangsbereich begrenzter Aufweitbereich, in dem es - wenn diese Pfanne aus Metall gefertigt wird - zu überelastischen, bleibenden Verformungen kommt.

Aufgabe der Erfindung ist es, die eingangs diskutierte Konstruktion für Hüftgelenkspfannen, deren Hülse aus Metall besteht, so abzuwandeln, dass bei Einpressen des Einsatzes bleibende Verformungen vermieden werden. Diese Aufgabe wird dadurch gelöst, dass die Bereiche geringerer Wandstärke durch Ausnehmungen von mindestens einer Stirnseite her in ihrer Höhe verkürzt und im Querschnitt Kreisringsektoren sind, die über Viertelkreise mit Radien (in mm) $0,5 \leq r \leq 1$ in die Bereiche grösserer Wandstärke übergehen, und dass ferner die Umfangslänge jedes Kreisringsektors mindestens das Vierfache des Uebergangsradius beträgt.

Mit den Bereichen geringerer Wandstärke werden über den ganzen Umfang verteilt Abschnitte mit erhöhter Flexibilität geschaffen, die durch die Ausnehmungen verstärkt wird, in welchen Abschnitten dann eine Aufweitung erfolgt; um diese Aufweitung nicht auf eine Mantellinie dieser Bereiche zu beschränken, sondern in Ebenen senkrecht zur Polachse der Hülse über den ganzen Winkel zwischen den begrenzenden Meridianebenen zu verteilen, sind die Bereiche als Kreisringsektoren mit zueinander parallelen Begrenzungen mit einer endlichen Ausdehnung in Umfangsrichtung ausgebildet, wobei diese endliche Ausdehnung eine gewisse Mindestlänge in Umfangsrichtung aufweist. Durch alle diese Massnahmen wird erreicht, dass sich die Hülse über den ganzen Umfang relativ gleichmässig verteilt aufweitet, wobei die Aufweitung in jedem Bereich geringerer Wandstärke über den endlichen Abschnitt mit parallelen Wänden verteilt ist, so dass die Grenzen einer elastischen Dehnung nirgends überschritten werden.

Die Stabilität der Hülse kann verbessert werden, wenn ihr Schlitz von Bereichen grösserer Wandstärke begrenzt ist. Weiterhin ist es für die Stabilität von Vorteil, wenn in Ebenen senkrecht zur Polachse der Hülse die Winkel zwischen den begrenzenden Meridianebenen der Bereiche grösserer Wandstärke jeweils ein Mehrfaches derjenigen zwischen den begrenzenden Meridianebenen der Bereiche geringerer Wandstärke betragen.

Ferner ist es vorteilhaft, wenn die Wandstärken der Hülse von ihrer Spitze zur Basis hin zunehmen, um dem zur Basis hin vergrösserten Durchmesser und damit vergrösserten Deformationsmoment Rechnung zu tragen. Die Weiterleitung der die Hülse belastenden Kräfte kann verbessert werden, wenn mindestens die den Schlitz begrenzenden und die diametral dazu liegenden Wandbereiche grösserer Wandstärke an der Basis mit nach aussen gerichteten Laschen versehen sind, wobei es sich für die Fixierung der Hülse zusätzlich als zweckmässig erwiesen hat, wenn die diametral gegenüber den Schlitzbegrenzungen liegenden Laschen mit Durchtrittsbohrungen für Knochenschrauben versehen sind.

Um bei Einpressen des Einsatzes die Aufweitung der Hülse über den zulässigen elastischen Bereich hinaus zu verhindern, kann man die Wandbereiche grösserer Wandstärke an der Spitze des Kegelstumpfes mit nach innen gerichteten Anschlägen für den Einsatz versehen; einzelne dieser Anschläge können darüberhinaus als Rotationssicherung für den Einsatz mit zur Basis weisenden Dornen bestückt sein, die in den aus Kunststoff bestehenden Einsatz eindringen.

Im folgenden wird die Erfindung anhand eines Ausführungsbeispiels im Zusammenhang mit der Zeichnung näher erläutert.

Fig. 1 ist eine Ansicht der neuen Hülse von der Kegelstumpfbasis her in Richtung der Rotationsache gesehen;

Fig. 2 gibt den Schnitt II-II von Fig. 1 wieder, während

Fig. 3 ein Detail aus Fig. 1 im vergrösserten Massstab zeigt.

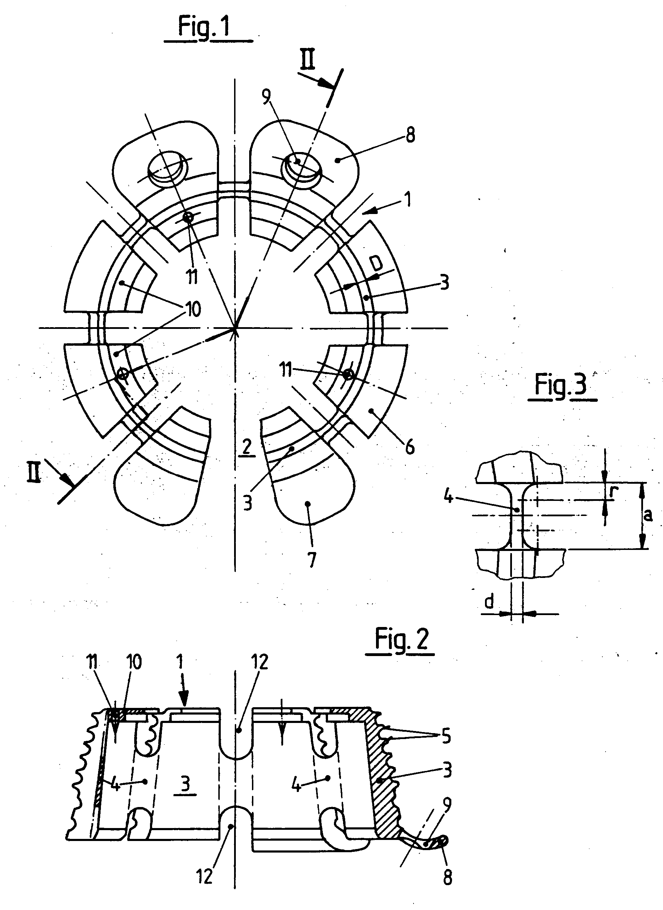

Die metallische Hülse 1 ist längs einer Mantellinie mit einem sich über ihre ganze Höhe erstreckenden Schlitz 2 versehen. Sie besteht in Umfangsrichtung alternierend aus Bereichen 3 grösserer Wandstärke D und Bereichen 4 geringerer Wandstärke d (Fig. 3). Wie Fig. 3 zeigt, ist ein Bereich 4 geringerer Wandstärke d aus einem Kreisringsektor a mit parallelen Wänden und zwei Radien r gebildet, die die Uebergänge zu den anschliessenden Bereichen 3 grösserer Wandstärke D "herstellen".

Die Radien r können dabei (in mm) Werte zwischen 0,5 und 1 annehmen, wobei zwischen der Länge a und den Radien r die Beziehung gelten muss: a $\geqq$ 4 x r.

Aus Fig. 2 ist zu ersehen, dass die Bereiche 4 geringerer Wandstärke d von beiden Stirnseiten des Kegelstumpfes her Ausnehmungen 12 aufweisen, durch die ihre Flexibilität erhöht wird. Bei einer Hülse aus Titan können die Wandstärken der Bereiche 3 und 4 etwa in der Mitte des Kegelstumpfes beispielsweise betragen: D = 2,5 bis 5 mm und d = 0,5 bis 2 mm.

Die Dicken d und D der Wand nehmen in jedem Bereich von der Basis zur Spitze des Kegelstumpfes hin ab; darüber- hinaus sind die Bereiche 3 grösserer Wandstärke D aussen durch in Umfangsrichtung verlaufende Rippen 5 mit einer Oberflächenstruktur versehen, die das Ein- und Anwachsen von Gewebe fördern soll.

Die Bereiche 3 grösserer Wandstärke D überdecken in Umfangsrichtung jeweils einen Kreissektor mit einem grösseren Winkel als die Bereiche 4 geringerer Wandstärke d. An ihrer Basis sind darüberhinaus nach aussen weisende Laschen 6, 7 und 8 angeordnet, von denen die den Schlitzen 2 begrenzenden Laschen 7 und die diametral gegenüberliegenden Laschen 8 gegenüber den Laschen 6 grossflächiger ausgebildet sind. Die Laschen 8 haben zusätzlich Durchtrittsbohrungen 9 für nicht gezeigte Knochenschrauben.

Die Laschen 6, 7 und 8 haben die Aufgabe, die Abstützung und Fixierung der Hülse 1 im Becken zu verbessern.

An seiner Spitze ist der Kegelstumpf in den Kreissektoren der Bereiche 3 grösserer Wandstärke D durch Anschläge 10 für den nicht gezeigten Kunststoff-Einsatz abgeschlossen, der die eigentliche Pfannenschale für die Aufnahme eines ebenfalls nicht gezeigten Gelenkkopfes enthält. Einzelne Anschläge 10 sind schliesslich mit Dornen 11 versehen, die beim Einpressen in den Einsatz eindringen und ihn gegen unbeabsichtigte Rotationen sichern.

**Patentansprüche**

1. Zweiteilige Hüftgelenkspfanne zur zementfreien Verankerung im Becken, bestehend aus einer einseitig geschlitzten, kegelstumpfförmigen Hülse (1) und einem die eigentliche Pfannenschale enthaltenden, im Passitz in den Innenhohlraum der Hülse (1) einpressbaren Einsatz, wobei die aus Metall gefertigte Hülse (1) in Umfangsrichtung Bereiche (3) grösserer Wandstärke (D) alternierend mit solchen (4) geringerer Wandstärke (d) aufweist, **dadurch gekennzeichnet,** dass die Bereiche (4) geringerer Wandstärke (d) durch Ausnehmungen (12) von mindestens einer der Stirnseiten her in ihrer Höhe verkürzt und im Querschnitt Kreisringsektoren sind, die über Viertelkreise mit Radien (r) (in mm) 0,5 $\leqq$ r $\leqq$ 1 in die Bereiche (3) grösserer Wandstärke (D) übergehen, und dass ferner die Umfangsfläche (a) jedes Kreisringsektors mindestens das Vierfache des Uebergangsradius (r) beträgt.

2. Hüftgelenkspfanne nach Anspruch 1, dadurch gekennzeichnet, dass der Schlitz (2) der Hülse (1) von Bereichen (3) grösserer Wandstärke (D) begrenzt ist.

3. Hüftgelenkspfanne nach Anspruch 1 oder 2, dadurch gekennzeichnet, dass in Ebenen senkrecht zur Polachse der Hülse (1) die Winkel zwischen den begrenzenden Meridianebenen der Bereiche (3) grösserer Wandstärke (D) jeweils ein Mehrfaches derjenigen zwischen den begrenzenden Meridianebenen der Bereiche (4) geringerer Wandstärke (d) betragen.

4. Hüftgelenkspfanne nach einem der Ansprüche 1 bis 3, dadurch gekennzeichnet, dass die Wandstärken (D,d) der Hülse (1) von ihrer Spitze zur Basis hin zunehmen.

5. Hüftgelenkspfanne nach einem der Ansprüche 1 bis 4, dadurch gekennzeichnet, dass mindestens die den Schlitz (2) begrenzenden und die diametral dazu liegenden Wandbereiche (3) grösserer Wandstärke (D) an der Basis mit nach aussen gerichteten Laschen (7,8) versehen sind.

6. Hüftgelenkspfanne nach Anspruch 5, dadurch gekennzeichnet, dass die diametral gegenüber den Schlitzbegrenzungen liegenden Laschen (8) mit Durchtrittsbohrungen (9) für Knochenschrauben versehen sind.

7. Hüftgelenkspfanne nach einem der Ansprüche 1 bis 6, dadurch gekennzeichnet, dass die Wandbereiche (3) grösserer Wandstärke (D) an der Spitze des Kegelstumpfes mit nach innen gerichteten Anschlägen (10) für den Einsatz versehen sind.

8. Hüftgelenkspfanne nach Anspruch 7, dadurch gekennzeichnet, dass einzelne Anschläge (10) mit zur Basis weisenden Dornen (11) bestückt sind.

## Claims

1. A two-piece acetabular cup for cement-free anchoring in the pelvis, comprising a frusto-conical sleeve (1) slotted on one side and an insert which contains the acetabular cup proper and can be pressed into the cavity in the sleeve (1) with a snug fit, the sleeve (1), which is of metal, having zones (3) of greater wall thickness (D) alternating in the peripheral direction with zones (4) of smaller wall thickness (d), characterised in that the zones (4) of smaller wall thickness (d) are shortened in their height by recesses (12) running from at least one of the end faces and are in cross-section annular sectors which merge into the zones (3) of greater wall thickness (D) by way of quadrants of radius (r) (in mm) $0.5 \leqq r \leqq 1$, and in that in addition the peripheral surface (a) of each annular sector is at least four times the transition radius (r).

2. An acetabular cup as claimed in claim 1, characterised in that the slot (2) in the sleeve (1) is defined by zones (3) of greater wall thickness (D).

3. An acetabular cup as claimed in claim 1 or 2, characterised in that in planes perpendicular to the polar axis of the sleeve (1) the angles between the defining meridian planes of the zones (3) of greater wall thickness (D) respectively equal a multiple of those between the defining meridian planes of the zones (4) of smaller wall thickness (d).

4. An acetabular cup as claimed in any of claims 1 to 3, characterised in that the wall thicknesses (D, d) of the sleeve (1) increase from apex to base.

5. An acetabular cup as claimed in any of claims 1 to 4, characterised in that at least those wall portions (3) of greater wall thickness (D) which define the slot (2) or are diametrically opposite to it are provided at the base with outwardly directed tongues (7, 8).

6. An acetabular cup as claimed in claim 5, characterised in that the tongues (8) situated diametrically opposite the slot boundaries are provided with bores (9) for the passage of bone screws.

7. An acetabular cup as claimed in any of claims 1 to 6, characterised in that the wall portions (3) of greater wall thickness (D) are provided at the apex of the truncated cone with inwardly directed stops (10) for the insert.

8. An acetabular cup as claimed in claim 7, characterised in that individual stops (10) are equipped with spikes (11) pointing towards the base.

## Revendications

1. Cavité cotyloïde en deux parties destinée à se fixer sans ciment dans le bassin et se composant d'un manchon tronconique (1) dont un côté est fendu et d'une pièce rapportée qui comprend la cavité cotyloïde proprement dite et qui se fixe à ajustement serré par enfoncement dans la cavité du manchon (1), le manchon (1), qui est réalisé en métal, comportant dans la direction de la circonférence des régions (3) de grande épaisseur de paroi (D) qui alternent avec les régions (4) de moindre épaisseur de paroi (d), caractérisée en ce que les régions (4) de moindre épaisseur de paroi (d) ont une hauteur raccourcie par des échancrures (12) réalisées sur au moins l'un de leurs côtés extrêmes et elles sont en coupe transversale des secteurs d'anneau circulaire qui forment la transition avec les régions (3) de grande épaisseur de paroi (D) par des quarts de cercle de rayon (r) (en mm) de $0,5 \leqq r \leqq 1$ et en ce que par ailleurs la longueur circonférentielle (a) de chaque secteur d'anneau circulaire est au moins le quadruple du rayon de transition (r).

2. Cavité cotyloïde selon la revendication 1, caractérisée en ce que la fente (2) du manchon (1) est délimitée par des régions (3) de grande épaisseur de paroi (D).

3. Cavité cotyloïde selon la revendication 1 ou 2, caractérisée en ce que les angles inscrits entre les plans méridiens délimitant les régions (3) de grande épaisseur de paroi (D) sont un multiple de chacun de ceux qui sont inscrits entre les plans méridiens délimitant les régions (4) de moindre épaisseur de paroi (d), dans des plans perpendiculaires à l'axe polaire du manchon (1).

4. Cavité cotyloïde selon l'une des revendications 1 à 3, caractérisée en ce que les épaisseurs de paroi (D, d) du manchon (1) croissent de sa pointe vers sa base.

5. Cavité cotyloïde selon l'une des revendications 1 à 4, caractérisée en ce qu'au moins les régions de parois (3) de grande épaisseur (D) qui délimitent la fente (2) et celles qui leur sont diamétralement opposées sont munies à la base de pattes (7, 8) orientées vers l'extérieur.

6.  Cavité cotyloïde selon la revendication 5, caractérisée en ce que les pattes (8) diamétralement opposées aux délimitations de la fente comportent des trous (9) de passage de vis à os.

7.  Cavité cotyloïde selon l'une des revendications 1 à 6, caractérisée en ce que les régions de paroi (3) de grande épaisseur (D) comportent à la pointe du tronc de cône des butées (10) orientées vers l'intérieur et destinées à la pièce rapportée.

8.  Cavité cotyloïde selon la revendication 7, caractérisée en ce que certaines des butées (10) comportent des pointes (11) orientées vers la base.

## Fig. 1

## Fig. 3

## Fig. 2